# EUROPEAN PATENT SPECIFICATION

(11) **EP 4 685 808 B1**
(45) Date of publication and mention of the grant of the patent: **15.07.2026**
(21) Application number: 25192190.4
(22) Date of filing: 28.07.2025
(51) Int. Cl.: G16H 15/00, G16H 30/40, G16H 50/20

(54) **A METHOD AND A SYSTEM FOR PREPARING A RADIOLOGY REPORT**
VERFAHREN UND SYSTEM ZUR ERSTELLUNG EINES RADIOLOGIEBERICHTS
PROCÉDÉ ET SYSTÈME DE PRÉPARATION D'UN RAPPORT DE RADIOLOGIE

(30) Priority: 26.07.2024 EP 24461603
(43) Date of publication of application: 28.01.2026
(73) Proprietor: Pixel Technology Sp. z o.o., 93-558 Lodz (PL)
(72) Inventor: MUSIALEK, Jakub, 93-558 Lodz (PL); PODYMA, Marek, 93-558 Lodz (PL); PUZIO, Tomasz, 93-558 Lodz (PL); WIECEK, Piotr, 93-558 Lodz (PL); DUNIKOWSKI, Kosma, 93-558 Lodz (PL); BIALKOWSKI, Sebastian, 93-558 Lodz (PL)
(74) Representative: Kancelaria Eupatent.pl Sp. z o.o.

(56) References cited:
- WO-A1-2018/236497
- WO-A2-2022/212771

## Description

### TECHNICAL FIELD

The present invention relates to a computer-implemented interface for implementing a method and a system for preparing a radiology report.

### BACKGROUND

Radiology reporting involves interpretation and description of radiology studies, such as X-rays, computed tomography (CT) scans, magnetic resonance imaging (MRI) scans, and other imaging modalities. These reports are critical for diagnosing and treating a wide range of medical conditions. However, the process of preparing these reports is time-consuming and labor-intensive, requiring a high degree of expertise and precision.

Radiologists must meticulously analyze each image, identifying and reporting every relevant feature. This process can be complicated by the sheer volume of images that must be reviewed for each patient, as well as the complexity and variability of the images themselves. Furthermore, the reports must be prepared in a manner that is clear and comprehensible to other healthcare professionals, adding another layer of complexity to the task.

In order to address these challenges, various methods have been proposed to facilitate the preparation of radiology reports. For instance, some systems provide templates or structured reporting formats to guide the radiologist in describing the images. These systems can help to standardize the reporting process and ensure that all relevant information is included. However, they still require the radiologist to manually input the information, which can be time-consuming and prone to error.

Typically, radiologists are reporting the study using their radiology workstation and a RIS (Radiology Information System). The workstation setup typically includes three displays: two displays for handling radiology software and one display for handling reporting software.

The radiologist may prepare the report by typing or dictating text. The radiologist browses through the study to recognize pathologies and then describes those pathologies to radiology software to prepare the final report. This approach requires radiologists to shift their gaze from one display (radiology images) to another (text). This approach has fundamental problems: firstly, radiologists switch focus from image to text and reporting takes a lot of time, secondly, information can be lost: the radiologist may forget the size of the measurements or even forget to describe a pathology that has been detected, especially in the scenario of multiple pathologies present in the study

Another approach for the radiologist is to dictate text and describe detected pathologies. Therefore, the radiologist browses through the study to recognize pathologies and simultaneously dictates the whole report. Therefore, the radiologist is constantly focused on the image. However, this approach also has disadvantages: the radiologist has to verify the final report, and there are no templates available that could facilitate the dictation work.

WO2022212771A2 describes a reporting interface in which artificial-intelligence algorithms propose image-based findings that are shown as on-screen "cards" or hyperlinks linking a report sentence to the relevant image location. Each proposed finding must be explicitly accepted, edited, or rejected by the radiologist before it is inserted into the report. A user must make a manual hyperlink between a text finding and a region of interest on the image. This approach does not auto-populate a complete draft report: the template remains empty until the user confirms individual AI candidates. Consequently, the radiologist must work through a potentially long queue of suggested lesions, with the attendant risk that relevant items are overlooked or dismissed in error. The procedure involves lots of manual actions and little automation when preparing and updating the report.

### SUMMARY OF THE INVENTION

There is a need for improved systems and methods for facilitating the preparation of radiology reports. Such systems and methods should reduce the work required by the radiologist while maintaining or improving the accuracy and comprehensiveness of the reports.

One aspect of the invention is a computer-implemented method for assisting a user in preparing a radiology report based on radiology images. This method involves generating an initial radiology report and presenting it via a user interface that includes at least one image panel for displaying the radiology image and a text panel with a description of the contents of the radiology image.

In a specific aspect, the invention relates to a computer-implemented method for assisting a user in preparing a radiology report from at least one input radiology image, the method comprising:
- processing the at least one input radiology image with at least one artificial intelligence module to detect anatomies and pathologies and to generate positional data and descriptive data related to the detected anatomies and pathologies;
- generating, automatically and prior to user review, an initial radiology report comprising, for every detected pathology and for at least one detected anatomy, collective positional data and descriptive data related to the detected anatomies and pathologies;
- displaying the initial radiology report via a user interface comprising at least one image panel displaying the at least one input radiology image with the detected pathologies and the at least one detected anatomy marked thereon and a text panel with a descriptive data related to the detected pathologies and the at least one detected anatomy;
- allowing user review of the initial radiology report, by receiving from the user a feature pointer input marking a feature at the image panel or at the text panel, wherein the panel via which the feature pointer input is provided is selectable by the user;
- receiving an action identifier to be performed on the feature defined by the feature pointer input, wherein the action identifier is selected from a group comprising: addition of a feature, modification of a feature and deletion of a feature; and
- updating the initial radiology report by performing the action corresponding to the received action identifier with respect to the feature defined by the feature pointer input and presenting an updated radiology report via the user interface.

Unlike prior art solutions, in which AI findings are merely queued for possible inclusion and must be accepted one-by-one by the user, the present invention automatically compiles, before any user interaction, a fully populated draft report that aggregates positional and descriptive data for every pathology detected by the AI modules and at least one clinically relevant anatomy. Displaying this auto-populated draft side-by-side with synchronized image overlays addresses the technical problem of cognitive overload and omission risk that radiologists face when shuttling between separate detection lists, image viewers and blank reporting templates. Thus, the claimed workflow provides several concrete technical effects:
- seamless, single-panel interaction: image and narrative remain dynamically linked, so panning, zooming or window/level adjustments keep all overlays and text references perfectly registered, eliminating disruptive context-switching;
- shorter reporting times: the radiologist edits or confirms a ready-made draft instead of building the report from scratch or accepting findings piecemeal, cutting average turnaround time;
- higher diagnostic reliability: because the initial draft already lists all detected pathologies, inadvertent omission of an AI-recognized finding can be greatly reduced;
- robust traceability and auditability: every textual item remains anchored to precise image coordinates throughout all manipulations, ensuring transparent provenance of clinical statements. An important advantage of this is that the user can work on united report which is either image or text. This unification allow to multilingual accessibility (the text layer can be easily translated and presented in any language).

The present invention integrates machine-learning detection, natural-language synthesis, and synchronized visualization within a single reporting workstation, thereby effecting a concrete improvement to computer-human interaction technology.

In one aspect, the invention includes extracting information on the diagnostic technique of the radiology images prior to generating the initial radiology report, using an artificial intelligence module and/or statistical algorithm. This feature provides the technical advantage of streamlining the report preparation process by automatically identifying the imaging technique, which can save time and reduce errors.

Another preferred embodiment involves determining the anatomy imaged on the radiology images prior to generating the initial radiology report, using an artificial intelligence module and/or statistical algorithm. The technical advantage here is the enhancement of report accuracy by ensuring that the anatomical structures are correctly identified and placed in the initial report, allowing to map any detected by or pointer by radiologist pathology to anatomical part of the body without a need of writing a full line of text containing pathology name and localization. Thereby allowing to report studies faster and allowing precise comparison between reports.

A further preferred embodiment includes extracting information on pathologies imaged on the radiology images prior to generating the initial radiology report, using an artificial intelligence module and/or statistical algorithm. This feature offers the technical advantage of improving the comprehensiveness of the report by ensuring that all relevant and highly visible pathologies are initially identified and included
The method also entails receiving from the user a feature pointer input that marks a feature at of the panels. Additionally, the method includes receiving an identifier of an action to be performed on the feature defined by the feature pointer input, where the identifier indicates one of the following actions: addition of a feature, modification of a feature, or deletion of a feature. The radiology report is then updated by performing the action corresponding to the received identifier of the action with respect to the received feature and presenting the updated radiology report via the user interface.

The system's ability to receive feature pointer input from a user (with action) via any one of at least two of the panels (in particular, via the image panel and the text panel) can streamline the process of preparing a radiology report, as the user selects the most optimal tool to indicate the feature pointer. The report is automatically updated based on the identifier of the action related to the selected feature, wherein the updated contents of the report are automatically indicated on all panels (in particular, simultaneously on the image panels and on the text panel), therefore the radiologist does not need to switch their gaze between the imaging display and the report generation display. Moreover, the amount of information to be provided by the radiologist is minimized as compared to prior art dictation systems since the radiologist only has to provide simple indicators of feature pointer and identifier of an action, which are then expanded in detail when generating the report by artificial intelligence (AI) modules, such as large language models (LLMs) and/or other natural language processing (NLP) techniques. For example, once the user points to a location in the radiology image and indicates the pathology (either by defining it with text or speech or selecting from a predefined or dynamically generated list), the system can generate information for the report, such as "severe compression at L2-L3 from the left side X,Y,Z".

The system provides a user-friendly interface that increases the comfort of work of the radiologist, thereby making their work more efficient and less error-prone. This also improves adoption rates for use of the system by new users. The system facilitates navigation from anatomy or pathology between image and text using X,Y,Z coordinates, since these components are interconnected.

The ability to receive information from both a radiologist and an AI module allows the system to be easily adaptable to a variety of clinical settings and user preferences.

The final radiology report includes comprehensive information for all pathologies, such as the location within anatomical structures, X,Y,Z, description, and measurements of each pathology, as well as the impressions, updating radiology report.

An additional preferred embodiment is where the step of receiving the feature pointer input comprises receiving an initial input via image panel. This embodiment provides the technical advantage of allowing precise feature marking and annotation, which enhances the detail and accuracy of the report. The pathologies are visible and interconnected through image panel, full reports or/and single findings.

Another preferred embodiment comprises receiving an identifier of an action indicating the addition of a feature from an artificial intelligence module and/or statistical algorithm trained to detect pathologies. The technical advantage of this feature is the facilitation of report preparation by automatically suggesting possible pathologies for inclusion in the report, thereby reducing the radiologist's workload.

A further preferred embodiment is where the step of receiving the identifier of an action includes receiving a voice command from the user through a speech recognition system or through text input via a keyboard or other forms of input. This embodiment offers the technical advantage of enabling hands-free interaction with the system, which can increase the efficiency and ease of use for the radiologist.

An additional preferred embodiment is where the step of updating the radiology report includes using an artificial intelligence large language model module to automatically generate descriptive text based on the received feature pointer input. This feature provides the technical advantage of enhancing the quality and consistency of the report text, as well as saving time for the radiologist.

A preferred embodiment of the invention further includes generating a final radiology report that also comprises comparing current radiology findings with previous studies. The technical advantage here is the provision of a comprehensive report that includes a historical comparison, which can be critical for tracking the progression of a patient's condition.

Another beneficial feature is the assignment of unique colors to each detected finding across all image and text views. Such consistent visual encoding provides intuitive clustering at a glance, substantially reducing visual search time and cognitive load, a clear ergonomic benefit recognized in the field. This also allows a user to quickly select features based on color for performing a quick action, such as deletion or acceptance.

A further preferred embodiment includes the feature that the contents of the image panel are correlated with the contents of the text panel such that when a feature is selected on the image panel, a corresponding text related to that feature, if present, is highlighted on the text panel and if a feature is selected on the text panel, a corresponding X,Y,Z of the image fragment is highlighted on the image panel. This facilitates finding correlation between the image and text fragments and selection of features by the user.

The method may also introduce a weighted relevance score to filter anatomical structures included in the initial draft report. By prioritizing clinically critical structures based on factors such as proximity to pathology, protocol relevance, or guideline-based significance, this scoring mechanism reduces false-alarm fatigue and directs the radiologist's attention effectively to the most clinically meaningful findings.

In a further advantageous aspect, the graphical overlays representing anatomical and pathological findings can be stored in image-space coordinates and dynamically re-mapped in real time during any viewport manipulation such as zoom or pan. This approach ensures that overlays remain precisely registered at a sub-pixel level with the underlying medical images, thereby preserving diagnostic reliability and accuracy irrespective of how the images are viewed or manipulated.

Furthermore, the invention relates to a computer-implemented radiology reporting system, comprising:
- at least one display device configured to concurrently render:
   - an image panel displaying at least one input radiology image; and
   - a text panel displaying descriptive data related to features detected in the at least one input radiology image;
- an input interface configured to receive user input from a user;
- a data storage that stores the at least one input radiology image and radiology reports; and
- a controller comprising at least one processor and a memory storing instructions that, when executed, cause the controller to:
   - process the at least one input radiology image with at least one artificial-intelligence module to detect anatomies and pathologies and to generate, for each detected anatomy or pathology, corresponding positional data and descriptive data;
   - automatically generate, prior to user review, an initial radiology report that aggregates, for every detected pathology and for at least one detected anatomy, the respective positional data and descriptive data;
   - present the initial radiology report on the display device by:
      - overlaying graphical markers derived from the positional data onto the image panel, and
      - displaying the descriptive data in the text panel,
   - wherein the image and text panels are dynamically linked so that selection of a feature in one panel highlights the corresponding feature in the other panel;
   - receive, via the input interface and through a panel selected by a user, a feature-pointer input that designates a feature appearing in the image panel or the text panel;
   - receive, in association with the feature-pointer input, an action identifier that indicates one of: addition of a feature, modification of a feature, or deletion of a feature; and
   - update the radiology report by performing the action indicated by the action identifier with respect to the designated feature and refreshing the image and text panels so that they remain synchronized.

These and other features, aspects and advantages of the invention will become better understood with reference to the following drawings, descriptions and claims.

### BRIEF DESCRIPTION OF DRAWINGS

The present invention is shown by means of example embodiments on a drawing, wherein:
Fig. 1 shows a schematic representation of the computer system for report preparation in accordance with a first embodiment of the system;
Fig. 2 shows a flowchart illustrating the steps of the method in accordance with a first embodiment of the method.
Fig. 3 shows a user interface screen with the initial version of the radiology report in accordance with the first embodiment of the report.
Fig. 4A shows a detailed content of the sagittal plane image panel with pathology marked.
Fig. 4B shows a corresponding text panel with initial information and pathology description.
Fig. 5 shows a zoomed-in functionality on a normal-sized image panel with feature delineation.
Fig. 6a shows an initial user interface showing overall and zoomed-in images with a text panel comprising a single type of pathology description.
Fig. 6b shows a user interface displaying amended text and corresponding enlarged image panel.
Fig. 6c shows a user interface indicating deletion of an incorrectly marked feature.
Fig. 6d shows an updated user interface after deletion of the feature from images and text panel.
Fig. 7 shows a schematic representation of the computer system for report preparation in accordance with a second embodiment of the system;
Fig. 8 shows a flowchart illustrating the steps of the method in accordance a the second embodiment of the method.
Fig. 9 shows an initial user interface showing overall and zoomed-in images with a text panel comprising a both types of pathology description.

### DETAILED DESCRIPTION

The following detailed description is of the best currently contemplated modes of carrying out the invention. The description is not to be taken in a limiting sense, but is made merely for the purpose of illustrating the general principles of the invention.

The present invention relates to a computer-implemented method for preparing a radiology report based on radiology images. The method involves a series of steps that utilize both user input and artificial intelligence (AI) to generate a comprehensive and accurate radiology report.

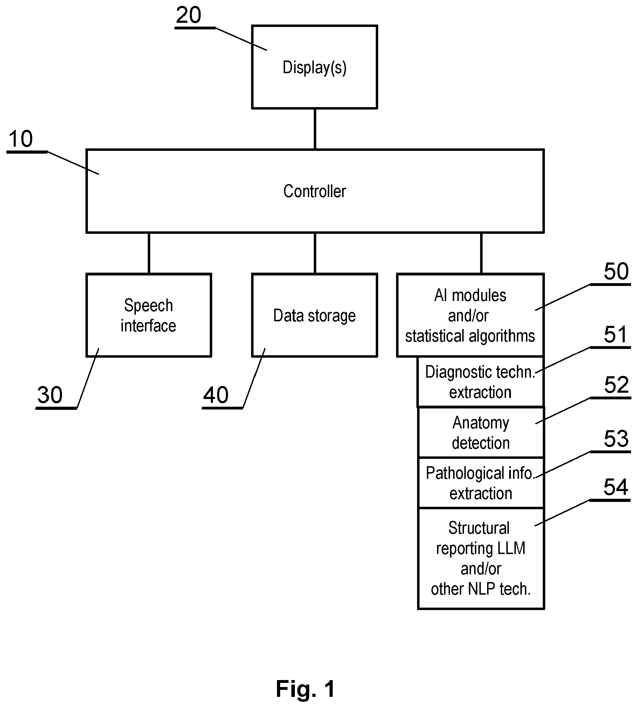

The method is implemented using a computer system, such as one in accordance with the first embodiment of the system, shown schematically in Fig. 1. The computer system comprises a controller 10 implemented as one or more computers configured to perform specific steps of the method described below. The steps may be performed on local computers, or at least some of the steps may be performed on cloud computing resources. Images and reports are presented to the user (the radiologist) via one or more displays 20. Data is received from and stored in a data storage 40, which can be connected to further data sources, such as imaging devices that provide various input diagnostic images or external databases. Apart from typical computer interfaces (keyboard, mouse, or 3D manipulators) the controller 10 is connected to a speech interface 30 that receives the user's speech and transforms the speech to text. Furthermore, one or more modules 50 can be present for autonomous realization of at least some tasks during generation of the report. The modules 50 can be artificial intelligence modules (operated by machine learning models) or statistical algorithms.

For example, the anatomy detection module 52 can be implemented as a three-dimensional convolutional neural network that combines a 3-D U-Net backbone with residual skip connections and squeeze-and-excitation attention blocks. Incoming DICOM volumes can be first resampled to an isotropic 1 mm voxel grid and intensity-normalized to a fixed Hounsfield (CT) or relative signal (MRI) range. Each volume can be then partitioned into overlapping 128 × 128 × 128-voxel tiles that form the input tensor to the encoder path. Down-sampling can be performed by strided 3-D convolutions (kernel = 3) followed by group-normalization and GELU activation. The decoder path mirrors the encoder and incorporates skip-concatenation of corresponding shallow-layer feature maps to recover spatial detail. SE blocks recalibrate channel weights so that the network can prioritize subtle anatomical textures over background tissue. The network can be supervised with multiclass Dice-Focal loss calculated over a plurality of anatomical labels (such as "right pulmonary artery", "L5 vertebral body").

The pathology extraction module 53 can be realized as a two-stage detector-segmenter pipeline. The first stage may employ a 3-D RetinaNet-style region-proposal network that slides a set of five anchor sizes (e.g. 8 mm to 64 mm) over the feature pyramid produced by the shared backbone. Each anchor may output a focal-loss-optimized probability for a plurality of pathology classes (e.g., "ground-glass nodule", "annular fissure", "infrarenal aneurysm"). Positive anchors can be passed to a second-stage refinement head comprising a deformable 3-D convolution block followed by a transformer-based mask decoder. The mask decoder generates voxel-level segmentations at native image resolution, improving delineation of irregular or elongated lesions such as aortic dissections or intracanalicular disc protrusions. Besides the binary mask, the can module automatically derive quantitative descriptors, such as maximum axial diameter, craniocaudal span, mean attenuation or T2 signal, calcification percentage and, for dynamic contrast studies, time-attenuation curves extracted from a per-lesion arterial/venous region of interest. For example, lesion growth can be estimated by non-linear diffeomorphic registration to prior studies retrieved from PACS, enabling automatic calculation of volume-doubling time. A LLM module 54 can be a transformer-based, decoder-only language model fine-tuned on a corpus of de-identified radiology reports and structured synoptic templates. The base architecture can follows the transformer/NLP family.

Training of the modules 52, 53 and 54 can be carried out off-line on a high-performance compute cluster equipped with multiple GPU nodes or CPU. Each module can be first "warm-started" from publicly available, broadly trained weights, such as MedicalNet for volumetric CNNs (modules 52 and 53) and a 7-billion-parameter generic language model for module 54, and then fine-tuned on a proprietary, de-identified data set that includes fully segmented 3-D studies with anatomy and pathology masks created by board-certified radiologists, paired prior-current study series with longitudinal labels for growth estimation, and template-based narrative reports mapped to structured entities. During fine-tuning, stochastic data-augmentation routines (random rotations, intensity shifts, elastic deformations) can be applied to the imaging data, whereas label-smoothing and curriculum-learning schedules are employed for the language model. Optimisation can use AdamW with a linear warm-up and cosine decay of the learning rate; mixed-precision FP16 training can shorten wall-clock time and reduce GPU memory footprint. Convergence can be monitored with 10-fold cross-validation, and the final checkpoint exhibiting the best mean Dice (modules 52/53) or highest BLEU/ROUGE-L score (module 54) on the validation folds can be selected for deployment.

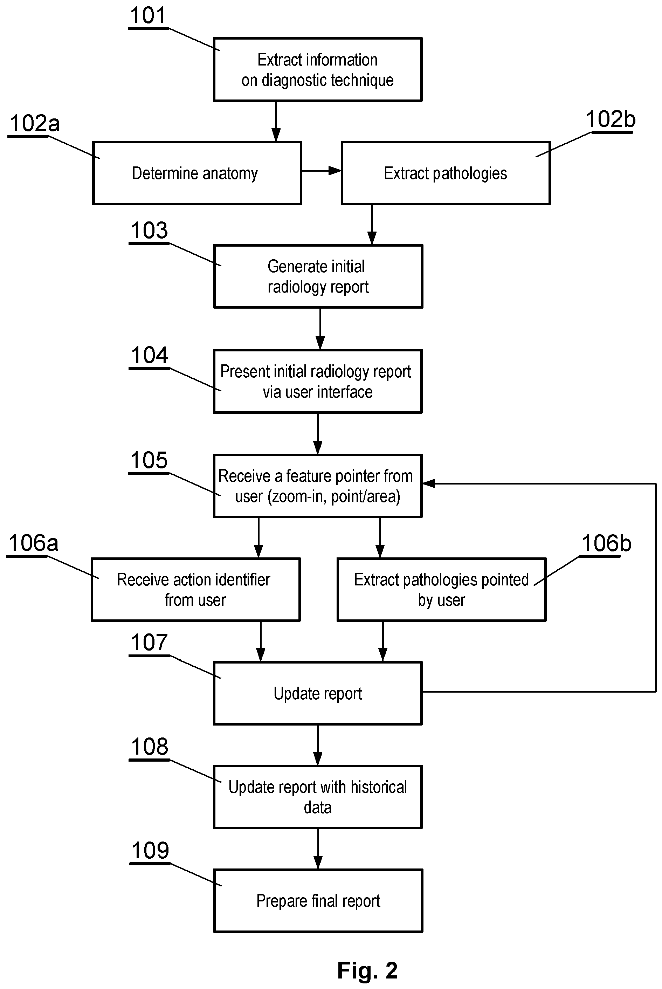

The method can be carried out according to the overall scheme presented in Fig. 2 in a first embodiment of the method.

Initially, in step 101, the procedure reads one or more input diagnostic images and extracts information on the diagnostic technique. For instance, the diagnostic image can be an X-ray image, a CT scan, an MRI scan, or other imaging modalities, preferably in a standard format such as DICOM. At least some information, such as the modality of the study, body part examined, protocol used for study acquisition, information about planes of acquisition, information about the contrast medium administration phases, sequences, radiation dose etc., can be extracted from the metadata of the DICOM file. Furthermore, a diagnostic technique extraction module 51 can be provided for determining additional information on the diagnostic technique.

In step 102a, an anatomy detection module 52 can analyze one or more input diagnostic images and extract information about the anatomy part, as well as detect any abnormalities. For instance, an AI module can be used that is trained to recognize various anatomy parts within the input diagnostic images. The anatomy detection module 52 outputs anatomy descriptors, including anatomy position data (such as coordinates of a point, voxel, area or volume within the input diagnostic image) and anatomy descriptive data (such as a brief description of a particular anatomy, e.g. S1 vertebra), for at least one anatomy detected within the image (preferably, for all anatomies detected within the image). This information, if relevant to the report, will be translated into narrative text and presented in the report. Examples for such relevant information are clinically important anatomical variants such as lumbarization of S1 vertebra or azygos lobe, etc. The anatomy detection module 52 can be configured to detect even very detailed fragments of anatomy, such as individual veins or nerves.

In step 102b, a pathological information extraction module 53 is used to extract pathological information from the image, such as the location of the pathology directly related to anatomical structures from step 102a, volumetric information about pathology, automated measurements are based on volumetry (for example cross, box/rectangle, circle/ellipse, irregular shapes), information on contrast enhancement (if relevant), information on signal on various MRI sequences (if relevant). The pathological information extraction module 53 outputs pathology descriptors, including pathology position data (such as coordinates of a point, voxel, area or volume within the input diagnostic image) and pathology descriptive data (such as a brief description of a particular pathology, e.g. bulging of intervertebral disc at L3/L4 level), for at least one pathology detected within the image (preferably, for all pathologies detected within the image) Multiple pathological information extraction modules 53 can be used in the system, each module 53 designed to extract pathologies related to a particular anatomy section. The system comprises multiple pathological information extraction modules 53, each configured to identify pathologies pertinent to designated anatomical sections.

The configuration of these modules 52, 53 extends beyond mere organ-level analysis, facilitating granularity or broader anatomical coverage as required. For instance, specific modules may be tailored for pathologies associated with the spine, heart, and lungs, respectively. Further specialization can be achieved through modules aimed at discrete anatomical structures within a broader category, such as modules for vertebral body analysis and spinal cord examination within the spinal category. Additionally, a module may be designed for example for comprehensive bone pathology detection across all osseous structures visualized in the imaging study, thereby enabling a spectrum of analysis from detailed to generalized anatomical assessments.

In step 103, an initial version of the radiology report is generated. The radiology report may comprise annotated radiology images (which are the initial diagnostic images with added metadata that describes specific detected features, such as marked points or boundaries corresponding to positional data output from steps 102a, 102b) and a text description (in a form of short descriptors corresponding to the descriptive data output from steps 102a, 102b and/or in a form of a narrative text generated by a LLM module 54, as will be explained later on).

The initial radiology report contains data related to every detected pathology and to at least one detected anatomy. Data related to each pathology include pathology positional data and pathology descriptive data, while data related to the at least anatomy include anatomy positional data and anatomy descriptive data. This data is aggregated into the initial radiology report prior to being filtered by any user input. By default, all detected pathologies are retained, while anatomies may be filtered for clinical relevance as explained below.

An anatomy is deemed as clinically relevant for inclusion in the initial radiology report when it meets predefined criteria. For example, the criteria may specify that the anatomy meets one or more of the following criteria:
- it spatially overlaps or neighbors at least one detected pathology, such as within a predefined distance such as <10 mm;
- relevance to examination protocol, for example the anatomy is mentioned in DICOM study description;
- guideline importance, for example it is included in a lookup table that defines anatomies commonly involved in critical findings for the scanned region, such as spinal canal in spine MRI, pulmonary arteries in chest CT;
- it attains a relevance score above a predetermined threshold that is calculated from modality, examination protocol or user-defined rules (e.g.;
- it is explicitly flagged by the radiologist in a preference profile, for example imported from prior examination results of the patient or a site-specific configuration file.

Anatomies that are detected in the image but do not satisfy the criteria can be omitted from the initial report to avoid information overload, while remaining available for later inspection if required.

Optionally, each criterion may have its weight and a total relevance score can be calculated and anatomies having a relevance score above a threshold can be included in the initial radiology report.

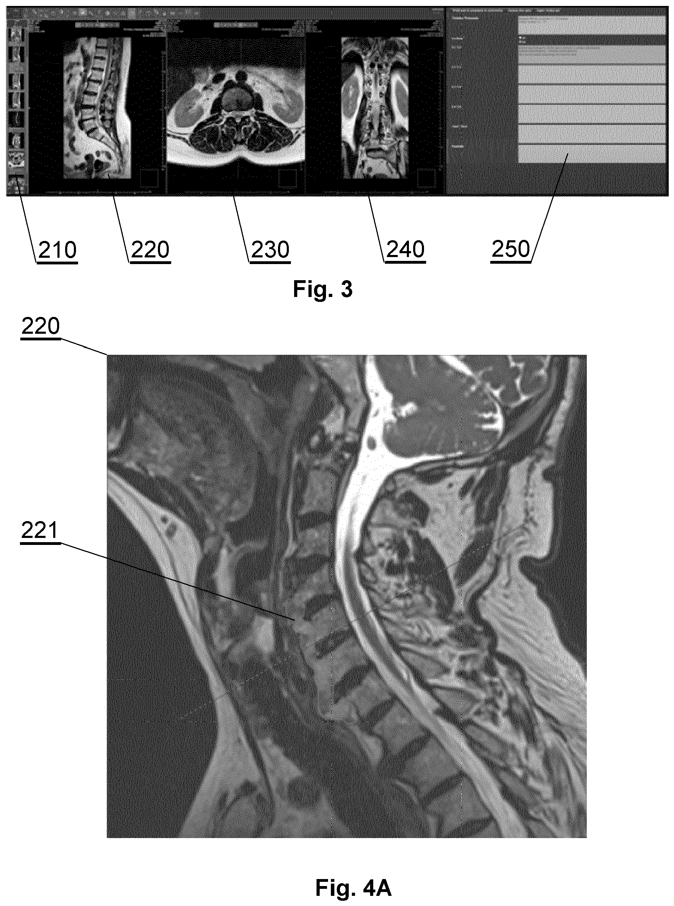

The initial version of the radiology report is presented in step 104 via a user interface screen, including annotated images in a simple 2D view or in three orthogonal planes, i.e. axial, sagittal and coronal, or 3D representation. The example interface shown in Fig. 3 includes a first panel 210 with a set of overview images, a second image panel 220 with a detailed image of a selected portion of anatomy in a sagittal plane, a third image panel 230 with the portion of the anatomy in an axial plane and a fourth panel 240 with the portion of the anatomy in a coronal plane, as well as a text panel 250 indicating the text contents of the initial report. The text panel 250 may be a stand-alone panel or a part of any image panel 210, 220, 230, 240, 224. Initially, the images and text contain information generated by the system in steps 102a, 102b, 103.

Each image panel 210, 220, 230, 240, 224 renders the original DICOM pixel data retrieved from PACS or local cache, rather than a down-sampled thumbnail. A layering engine in the graphics pipeline composites the diagnostic image in the bottom layer with one or more transparent overlay layers that contain pathology markers, anatomy boundaries and measurement graphics. Because the raw pixel grid is preserved, the radiologist retains access to the complete bit depth and can freely adjust window/level, magnification and pan position without losing fidelity.

For instance, the images contain markers indicating pathologies and the text contains a description of the pathology in a short form (as output from the pathology extraction module 54 or the VLM 55) or in a narrative form (as transformed by the LLM 54 or output from the VLM 55). In addition, the system may allow the user a selection of a language for presenting the textual descriptions. For example, the texts generated by the modules 52, 53, 55 can be generated initially in a default language (e.g. English) and if the language selected by user is a different language (e.g. German) then the LLM 54 or VLM 55 can be trained to translate specific short or narrative descriptors to the other language and present it in the text panel in the translated form.

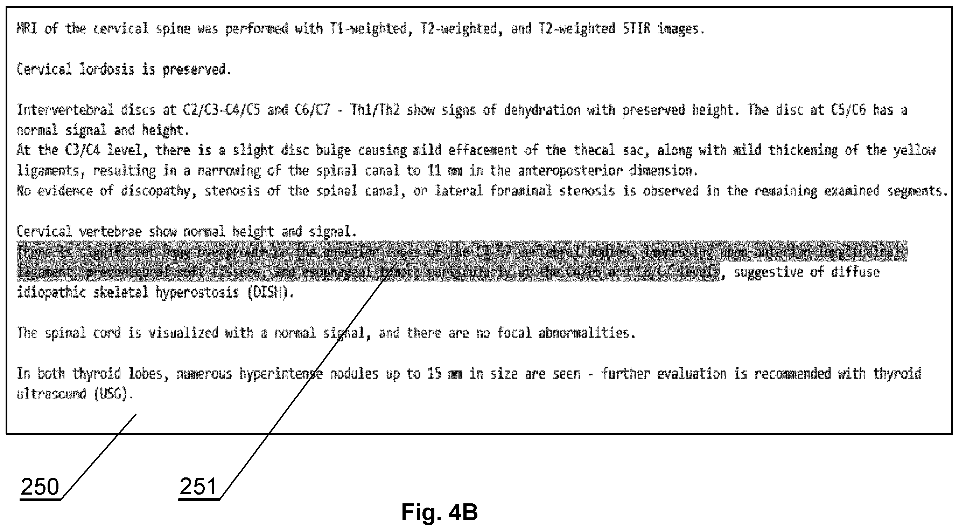

Fig. 4A shows an example of the detailed content of the sagittal plane image panel 220 including an image of a spine with a pathology 221 marked thereon. Fig. 4B shows an example of a corresponding panel 250 with text contents including the initial information generated by the system, including an overall description of the image and in particular a text description 251 of the pathology 221.

In step 105, the system receives, from an expert user, a feature pointer input that marks a feature at one of the image panels 210, 220, 230, 240, 224 or at the text panel 250. Specifically, the user can mark (by using an input interface such as a mouse, a keyboard, a 3D interface, an eye tracking system) a particular feature (such as a point or an area) at the image panel or at the text panel. The feature pointer input in the image panel may correspond to indication of a specific point (which may result in selection of that point or a pathology or anatomy which has been recognized by the modules 52, 53 at this point) or to encircling an area or volume (e.g. to mark a new space corresponding to e.g. a pathology that has not been recognized by the module 53). The feature pointer input in the text panel may correspond to indication of a specific point in the text (which may result in selection of a word in a vicinity of that point) or selection of a fragment of the text (e.g. a few words or a full sentence). This step allows the user to identify potential features of interest or concern within the medical images and provides initial pointers to these features (such as anatomies or pathologies or their detailed parameters). In this step the user may:
- point at new features which were not marked so far by the system in an autonomous procedure of steps 102a, 102b, in order to add new information to the report - in this case, the user will typically point at the image;
- point at the features marked so far by the system, in order to modify the information about these features - in this case, the user may point either at the image or at the text;
- point at the features marked so far by the system, in order to delete information that is considered by the user as erroneously generated - in this case, the user may point either at the image or at the text.

Next, in steps 106a, 106b the system receives an identifier of an action to be performed on the feature defined by the feature pointer input, wherein the action identifier may indicate one of the following actions: addition of a feature, modification of a feature or deletion of a feature.

Step 106a relates to receiving an action identifier from the user. For example, the user may provide detailed information about pathology type (such as: nodule, focal lesion, disc bulge, modeling of the dural sac), in order to describe a newly indicated feature or modify the description of the existing feature. Alternatively, the user may issue a short command such as "delete" in order to delete the previously marked feature. Preferably, in step 106a the user provides the additional information by speech, but other means can be used as well, such as typing via a keyboard. The action identifier can be obtained from explicit voice or keyboard commands, or implicitly from the context: for example, drawing a region of interest that has not been identified in the initial report yet can be interpreted as an add action, over-typing an existing text can be interpreted as a modify action, and pressing a "delete" hot-key can be identified as a deletion function.

Alternatively, or in addition to step 106a, an action related to addition of a new feature can be generated autonomously in step 106b by the system using the pathological information extraction module 53 that is instructed to analyze the feature pointed at by the user and describe a corresponding pathology.

In step 107, the report is updated by a set of findings delivered from the images or voice prompts, text according to the feature pointer input received in step 105 and the action identifier received in step 106a and/or 106b. Namely, the report is updated by adding a new feature with a corresponding description, modifying a description of an existing feature or deleting a previously existing feature. Therefore, each change triggers an immediate regeneration of the image data and descriptive data, as well as the narrative text, so that the image panel(s) and text panel remain fully consistent with the updated feature set.

The procedure can be performed repeatedly until the user does not wish to enter any more findings, modifications and then the procedure proceeds to step 108, wherein the report can be updated with information about historical studies of that patient.

In step 109, a final report is generated. The final report contains one or more of: identification of radiologic examination technique, overall information about patient anatomy, pathologies found in the examination, summary section.

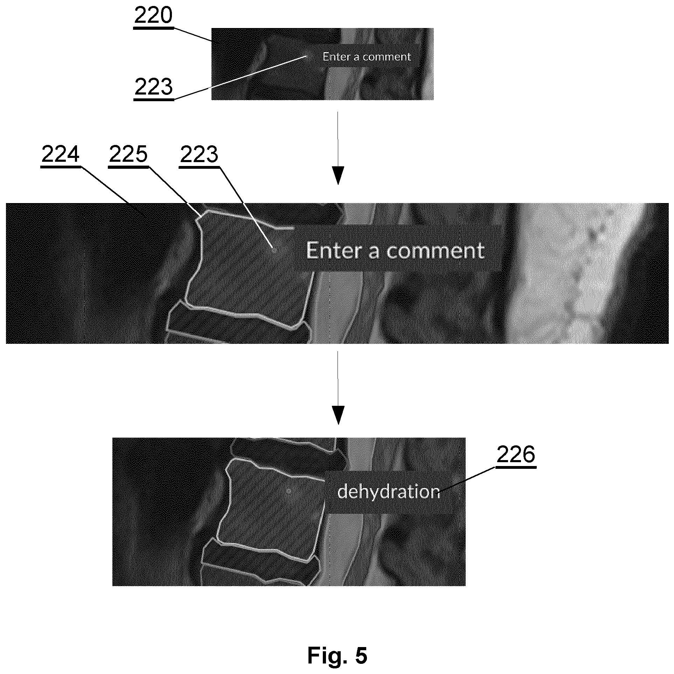

Optionally, as shown in Fig. 5, when a user points at a normal-sized image panel 220 at a feature 223 in step 105, the system may allow a zoom-in functionality to enlarge the surroundings of the feature 223 and generate a zoomed-in representation image panel 224. On that representation, the user may delineate an area 225 or instruct the anatomy detection module 52 to detect an area of interest at the pointed feature 223. If the area is detected by the module 52, the user may have an option to correct it manually. The pathology extraction module 53 can then make corresponding calculations of the volume and other measurements. As the user provides additional speech or text input in step 106a, the detected spoken feature may be immediately presented as a description 226 on the image, before a more detailed description is generated in the text report.

In addition, in step 108, if the currently reported study is associated with prior studies, then information from these prior studies can be added to the report generated in step 107, for example, to explain a change in the pathology between the current study and prior-N studies or prior-N to prior study, so that more of the relevant information is contained in the final report of step 109. Then, using registration/transformation algorithms, the corresponding pathologies can be located prior to the current and all the above combinations. Then subtraction or differentiation can be performed to provide a difference between pathology volume or measurements between the prior studies and the current study.

The full text information in the report can be generated in steps 103, 107, 108, 109 by a system such as Large Language Model (LLM) and/or other NLP (Natural Language Processing) techniques module 54 based on the basic information generated or provided in steps 101, 102a, 102b, 103, 105, 106a, 106b.

It should be noted that when the user interface is displayed to the user, the panel 250 indicating text contents is dynamically linked with the image panels 220, 230, 240 such that when a feature is selected (either by pointing to one of the image panels 220, 230, 240 or highlighting a fragment of text within the panel 250), it is highlighted both on the image panels 220, 230, 240 and in the panel 250 with the text. Therefore, the user is able to quickly and intuitively select a feature on the image panel 220, 230, 240, 250 that is most convenient to the user. Any additions, modifications or deletions of features are updated on all the panels 220, 230, 240, 250.

Each overlay object stores geometry in image-space coordinates (such as row, column, slice index or 3-D voxel index. At rendering of the user interface, the controller 10 multiplies these coordinates by the current viewport transformation matrix (comprising pan offset, zoom factor and any window-level LUT) to obtain screen-space positions. The mapping can be recalculated at the display frequency, so markers follow the image content during rapid drag or scroll operations. The same transform is applied when the user activates the zoom-in panel 224 (as in Fig. 5), ensuring that the enlarged representation shows correctly scaled and positioned markers.

In addition, other steps can be employed, such as overall analysis of the patient, such as mapping current measurements to a centile measurements database for the specific age, gender, and ethnicity of the patient. More specifically, in case of examination of the spine, the system may measure the distance between two spaces (for example, distance between intervertebral discs) and compare them to each other and optionally to a standard centile database, analyze cauda equina, verify with standard centile database, identify if an organ, bone, or recognizable part is in the boundaries of the centile database. By using the centile database, the system may create reports with measurements, perform triage and create a text report out of this analysis.

For example, a radiology report related to a patient's spine exam may include:
1. Radiology exam technique used: T1-weighted, T2-weighted, T2-weighted STIR. An AI or algorithm may recognize the protocol for the study using an image or study description, or an AI or algorithm may analyze the content of the series and determine whether the study was T1-weighted, T2-weighted, T2-weighted STIR with or without contrast.
2. Anatomical description. (for example: *Lumbar lordosis is preserved. The spinal cord and cauda equina are of the normal signal. The remaining intervertebral spaces show no signs of intervertebral disc herniation).*
3. Report on a set of pathological changes, such as:
   - L4/L5 modeling of the dural sac
   - L5/S1 disc height decreased
   - L5/S1 canal AP dimension of 11 mm, stenosis
   - L5/S1 disc dehydration
   - S1 modeling dural sac and nerve roots
   - Th12/L1 disc bulging
   with an overall image showing the precise locations of pathologies (this part of image presentation is optional)
4. A summary, generated by an AI model, preferably by the radiologist themselves.

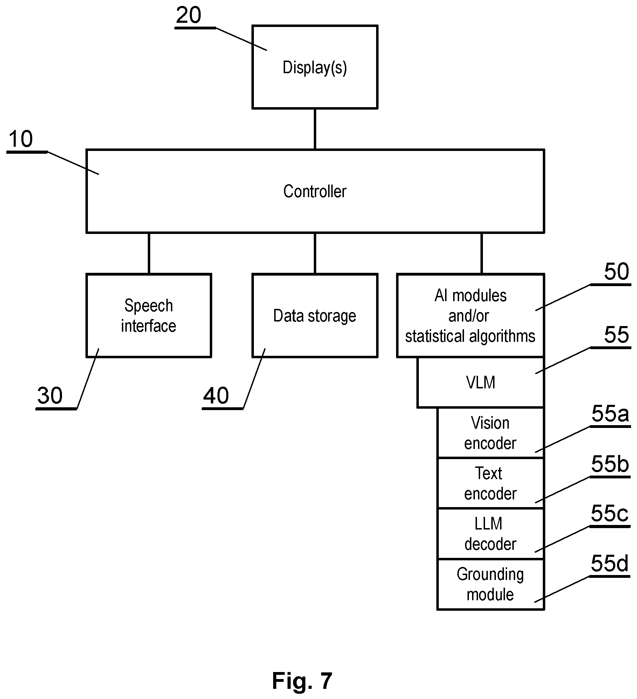

Alternatively, the method can be implemented using a computer system, such as one in accordance with the second embodiment of the system, shown schematically in Fig. 7. It is similar to that shown in Fig. 1, with the following differences. Instead of modules 52-55 it contains a single Vision Language Model, VLM 55. The VLM 55 comprises a vision encoder 55a, a text encoder 55b, a LLM decoder 55c and a grounding module 55d. The VLM is trained to receive, as input, one or more input diagnostic images and provide, as output, anatomy descriptors, pathology descriptors and optionally narrative text, as described so far.

Specifically, the VLM module 55 can be capable of:
- Automatically generating machine-readable description (report) for both anatomy and pathology. Each descriptor may includes categorical labels, precise positional data in the form of bounding boxes or voxel-level masks aligned to patient anatomical coordinates, and associated quantitative metrics, such as volumes, average Hounsfield units, or MRI signal intensities;
- Predicting additional metadata related to the imaging study itself through zero-shot or few-shot inference. This metadata may include information such as imaging modality type, contrast enhancement phases, and protocol compliance indicators;
- Optionally, producing a coherent narrative report in natural language. This narrative provides a human-readable summary of findings, structured similarly to traditional radiological reports.

The internal structure of the VLM module 55, depicted schematically in Fig. 7, comprises four interdependent neural network components that interact via a unified cross-modal representation space.

The vision encoder 55a processes input DICOM imaging data, either as individual 2-D slices or consolidated 3-D volumetric blocks, and converts these into a structured sequence of visual tokens. For example, input data undergoes standardized preprocessing to normalize pixel intensity values into consistent Hounsfield units or MRI signal ranges and to apply window-level adjustments. Subsequently, the processed data can be partitioned into uniform 3-D patches, each of dimension 16×16×16 voxels, and projected into a high-dimensional embedding space. These embedded patches can be then encoded by a robust 24-layer Vision Transformer (ViT-3D L), which captures spatial context through both absolute spatial positional embeddings (xyz coordinates) and additional slice-specific positional markers, ensuring accurate representation of the anatomical structures across the study.

The text encoder 55b ingests available textual inputs, such as patient history or descriptive metadata related to the imaging study. It can transform this textual content into linguistic tokens. For example, to achieve accurate contextual understanding, this encoder can employ a 12-layer RoBERTa-based architecture, fine-tuned specifically on an extensive dataset comprising over ten million radiology reports.

The LLM decoder can be responsible for generating natural-language narratives conditioned upon both visual and textual tokens. For example, it can utilize a powerful 16-layer GPT-style transformer architecture, that dynamically integrates visual information with linguistic context through cross-attention mechanisms. Furthermore, during its training phase, this module can be optimized to predict masked pathology terms, enhancing its capability to accurately describe anatomical and pathological features.

Finally, grounding module 55d serves as a linkage between visual tokens and their corresponding textual descriptions. Employing two dedicated cross-modal transformer layers, this module precisely projects visual tokens back onto the original imaging space and aligns them with generated linguistic tokens. It can produce spatially explicit outputs in the form of 3-D voxel-level masks or axis-aligned bounding boxes, along with numerical confidence scores. Additionally, the grounding module can include an optional regression mechanism for computing and associating quantitative measures, such as volumes and dimensional extents, with the identified features.

Collectively, these four modules 55a-55d operate within the integrated architecture of the VLM module 55, ensuring accurate and comprehensive interpretation of radiological imaging data, efficient extraction of clinically relevant information, and streamlined generation of structured and narrative radiology reports.

The vision language model 55 can be trained in three successive phases on a secure compute cluster with GPUs. Phase 1 (uni-modal pre-training) can initialize the 3-D Vision-Transformer encoder with self-supervised masked-patch prediction on a plurality of anonymized CT/MRI volumes (e.g. RadImageNet-3D) while the text encoder/decoder is warm-started from a generic LLM. Phase 2 (cross-modal alignment) can jointly optimize image and text tokens using a CLIP-style contrastive loss plus a masked-language-modelling objective, leveraging image-report pairs drawn from e.g. MIMIC-CXR, CheXpert, or institutional PACS archives and automatically generated synthetic captions that preserve Protected Health Information (PHI) redaction. Phase 3 (task-specific fine-tuning) can add supervised heads for voxel-level grounding (Dice/Focal loss), pathology classification (cross-entropy) and narrative generation quality (reinforcement learning from radiologist feedback with a custom reward for factual consistency).

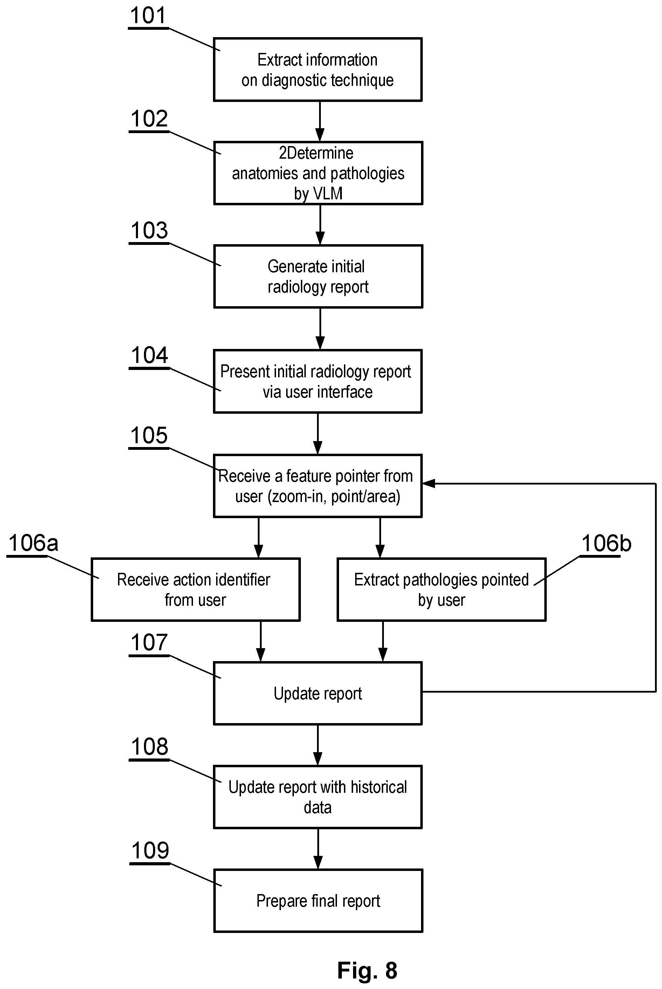

The computer system of the second embodiment operates in accordance with the procedure shown in Fig. 8, which is similar to that shown in Fig. 2, with the following differences. Instead of steps 101, 102a, 102b it comprises a single step 102 that corresponds to a single operation of the VLM 55, that produces data sufficient to generate the initial version of the radiology report in step 103.

### First example

Assume the MRI study of cervical spine contains a specific C3/C4 disc bulging that is not recognized by the AI modules and/or statistical algorithm. In that case, once diagnostic images are received, in step 101, the system will extract basic information about the diagnostic technique. Next, in step 102a the system will determine the anatomy as a cervical spine. In step 102b the system may return no specific pathologies. Alternatively, a VLM 55 detects anatomies in step 102. An initial report generated in step 103 and presented in step 104 will therefore include basic information about the diagnostic technique (such as MRI) and about the anatomy part (such as a cervical spine) along with information that no abnormalities were found. Then, in step 105 the user (radiologist) may simply point at the sagittal plane image panel 220 to a position between C3/C4 vertebrae and do not provide any further information, in order to initiate operation of the pathology extraction module that will compare that fragment against internal database with similar pathologies providing output with greater scrutiny and in step 106b recognize the disc bulging, along with determination of its volume and measurements. Therefore, the text report will be updated in step 107 with additional information about that bulging and the bulging will be marked on the image panels 220, 230, 240. In step 109 a final report with that information can be generated.

### Second example

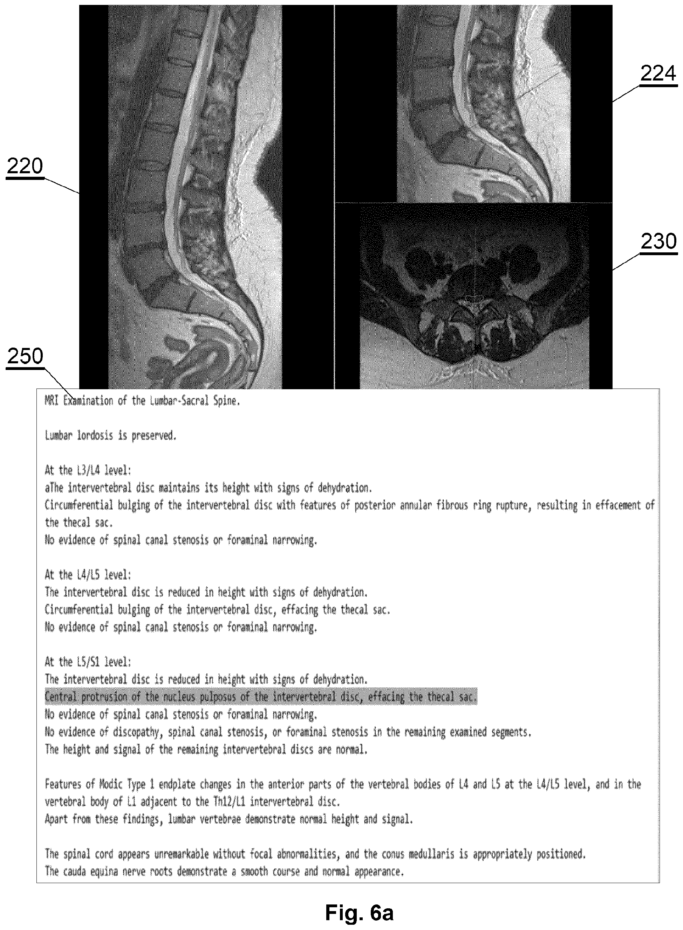

This example will present a standard process of annotating a diagnostic image. In step 101, an MRI image of a spine is received and information on diagnostic technique (MRI) is extracted by the diagnostic technique extraction module 51. In step 102a the anatomy detection module 52 detects that the diagnostic image contains a spine and detects its vertebrae. In step 102b, the pathological information extraction module 53 determines details of pathologies detected. Alternatively, a VLM 55 detects anatomies and pathologies in step 102. Consequently, the initial radiology report generated in step 103 and initial user interface generated in step 104, as shown in Fig. 6a, includes basic information generated by the modules 51-53 and converted to a descriptive text by the system such as LLM (Large Language model) and/or NLP (Structural Reporting NLP) module 54. In that case, the interface has been configured to show an overall image of the sagittal plane image panel 220, an overall image of the axial plane 230 and a zoomed-in representation image panel 224 of the sagittal plane, as well as a text panel 250. The user interface allows the user to point at a selected feature either at one of the image panels 220, 230, 224 or at the text panel 250, such that when a feature is selected at one of the elements, it is highlighted on all other elements. In this case, a central protrusion and its corresponding description are highlighted.

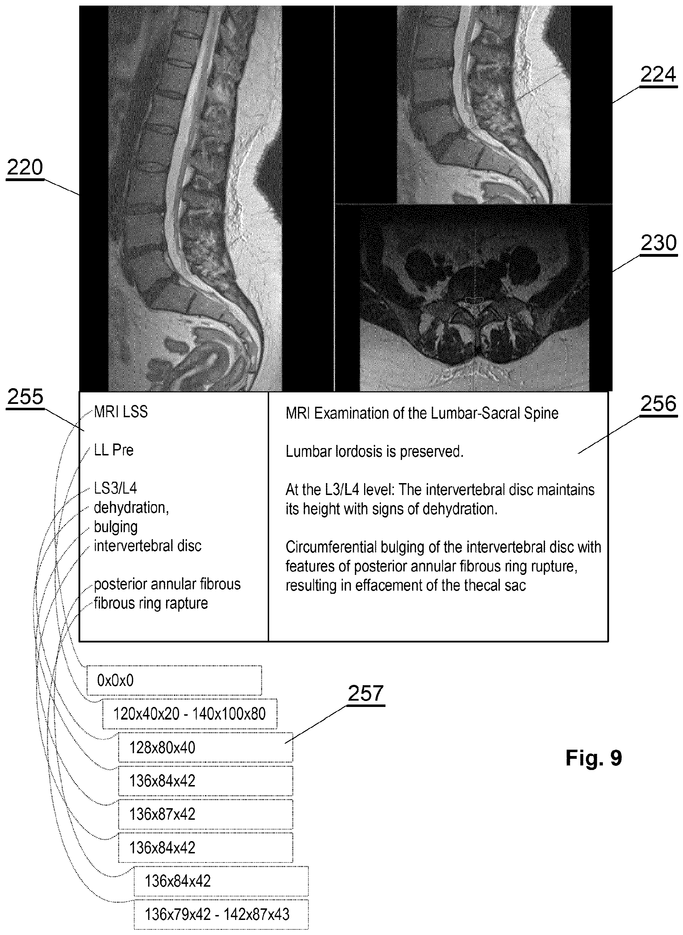

Fig. 9 shows a user interface similar to that of Fig. 6a, wherein instead of a single-type description text panel 250 there are shown two text panels 255, 256 - the first text panel 255 showing short descriptors of the pathology along with corresponding anatomy (e.g. LS3/L4; dehydration; bulging; intervertebral disc) and the second text panel 256 showing narrative descriptors of the pathology (e.g. at the L3/L4 level: The intervertebral disc maintains its height with signs of dehydration) as generated by the LLM module 54 or the LLM decoder 55c of the VLM 55. Preferably, text fragments corresponding to the same anatomy or pathology are presented in both text panels 255, 256 with the same color (unique for each pathology) or the same background (unique for each pathology). Corresponding colors can be used to mark points or areas/volumes in the image panels 220, 230, 224 related to the particular anatomy or pathology. Fig. 9 also shows schematically positional (spatial) data 257, which are not displayed as such, but stored in memory as indicators of xyz position or area and used to mark the corresponding features (anatomies or pathologies) in one or more of the image panels.

The color assignment can be performed based on a lookup table keyed by a unique feature identifier. When the radiologist adds, edits or deletes a feature, the module can update the table and propagate the color to both text panels and to any image overlay in real time. If a feature is deleted, its color is freed and may be reassigned to a newly added feature, ensuring the palette remains visually distinct. When an initial radiology report is created, a default lookup table can be used with standard colors assigned to typical pathologies, so that initially each pathology of this type is marked with the standardized color, so that the radiologist can have an intuitive overview of the identified pathology types just by looking at the colors of the initial radiology report.

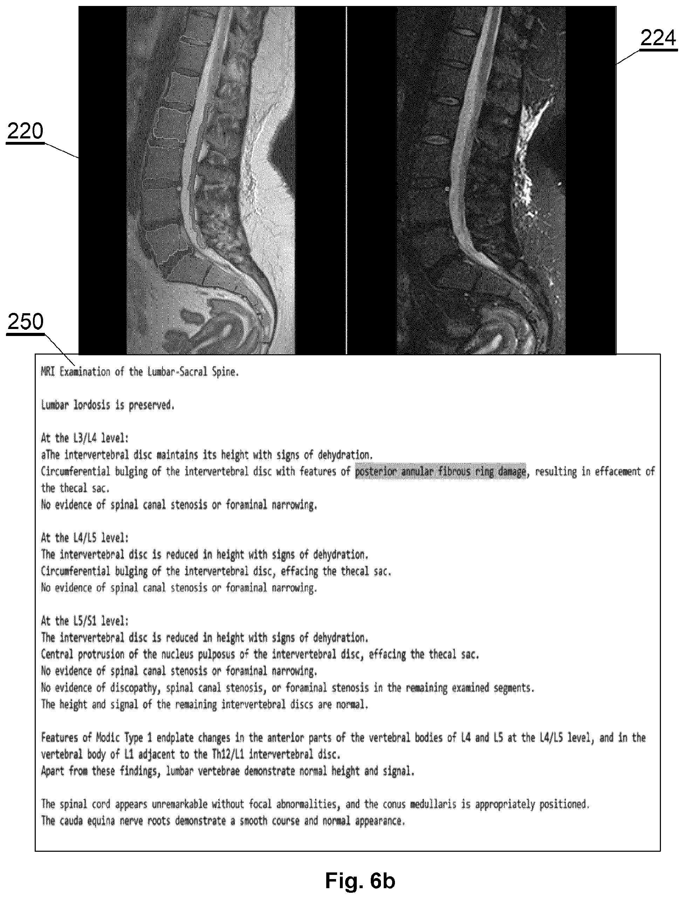

Subsequently, the physician may determine that the autonomously detected feature of "posterior annular fibrous ring rupture" is incorrectly described, and therefore may mark this feature in step 105 on the text panel 250 and provide in step 106a a keyboard input to amend it to "posterior annular fibrous ring damage". As the feature is being amended in the text panel, its enlarged representation can be displayed in the zoomed-in representation image panel 224 (which is further enlarged such as to cover the previously displayed image of the axial plane image panel 230), as shown in Fig. 6b.

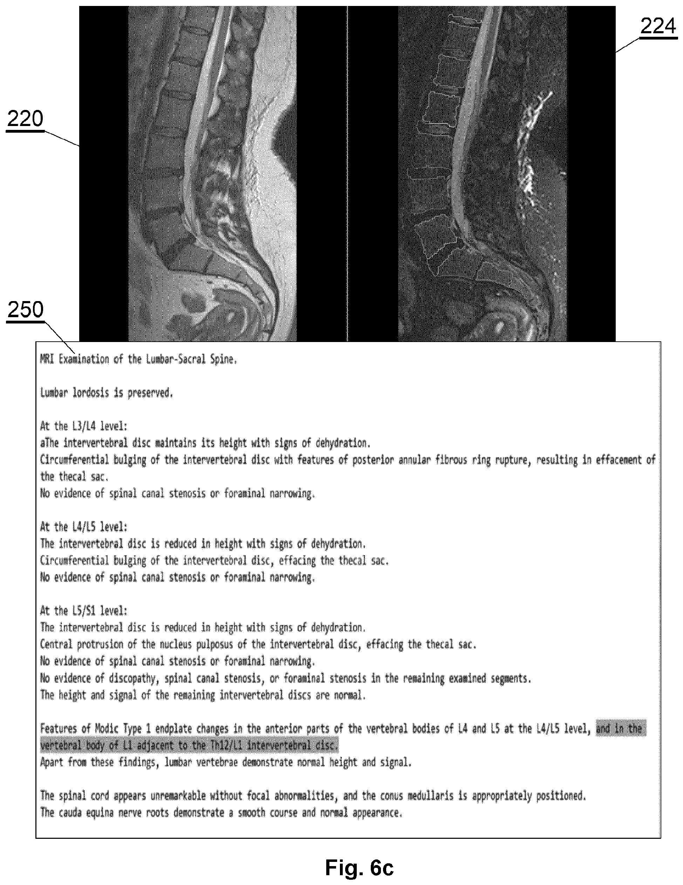

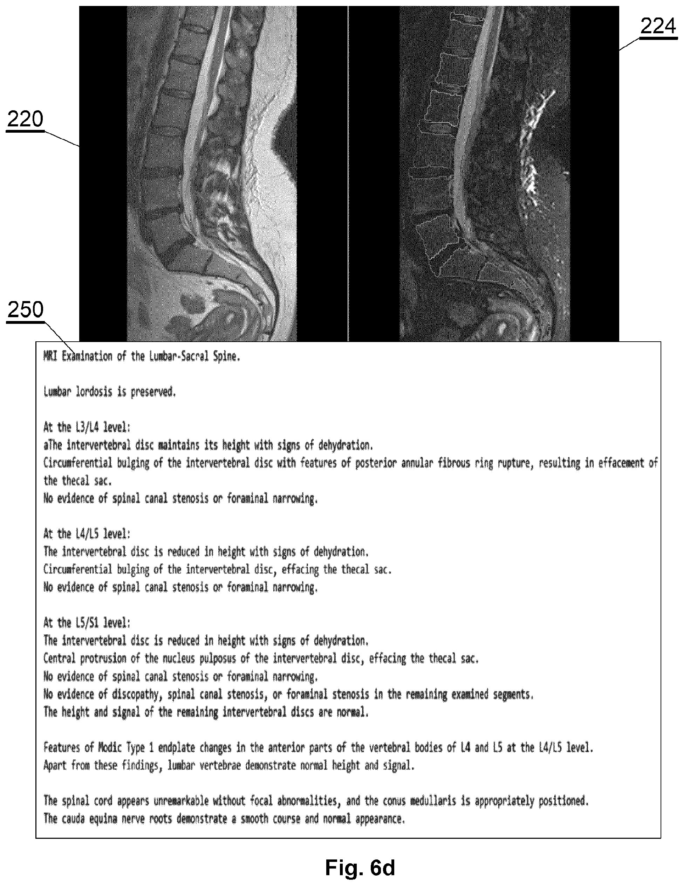

Next, the physician may recognize that the pathological information extraction module 53 has incorrectly marked endplate changes lesion located in Th12/L1. The physician can mark this feature in step 105 on the text panel 250 or on one of the images and provide in step 106a a keyboard input (such as pressing a "delete" key) or speech input (such as saying "delete") to delete this feature, as shown in Fig. 6c. Consequently, the feature will be deleted from the images and the text panel 250, as shown in Fig. 6d.

If no other changes are to be made, the system may be instructed to generate a final report in step 109.

While the invention has been described with respect to a limited number of embodiments, it will be appreciated that many variations, modifications and other applications of the invention may be made. Therefore, the claimed invention as recited in the claims that follow is not limited to the embodiments described herein.

## Claims

1. A computer-implemented method for assisting a user in preparing a radiology report from at least one input radiology image, the method comprising:
- processing (102, 102a, 102b) the at least one input radiology image with at least one artificial intelligence module (52, 53) to detect anatomies and pathologies and to generate positional data and descriptive data related to the detected anatomies and pathologies;
- generating (103), automatically and prior to user review, an initial radiology report comprising, for every detected pathology and for at least one detected anatomy, collective positional data and descriptive data related to the detected anatomies and pathologies;
- displaying (104) the initial radiology report via a user interface (20) comprising at least one image panel (210, 220, 230, 240, 224) displaying the at least one input radiology image with the detected pathologies and the at least one detected anatomy marked thereon and a text panel (250, 255, 256) with a descriptive data related to the detected pathologies and the at least one detected anatomy;
- allowing user review of the initial radiology report, by receiving (105) from the user a feature pointer input marking a feature at the image panel (210, 220, 230, 240, 224) or at the text panel (250, 255, 256), wherein the panel (210, 220, 230, 240, 224, 250, 255, 256) via which the feature pointer input is provided is selectable by the user;
- receiving (106a, 106b) an action identifier to be performed on the feature defined by the feature pointer input, wherein the action identifier is selected from a group comprising:
addition of a feature, modification of a feature and deletion of a feature; and
- updating (107) the initial radiology report by performing the action corresponding to the received action identifier with respect to the feature defined by the feature pointer input and presenting an updated radiology report via the user interface (20).

2. The method according to claim 1, further comprising, prior to generating (103) the initial radiology report, performing at least one of:
- extracting (101) information on diagnostic technique of the at least one radiology image, using an artificial intelligence module and/or statistical algorithm (51);
- determining (102a) at least one anatomy imaged on the at least one input radiology image, using the artificial intelligence module (52), which is trained to detect anatomies, and/or a statistical algorithm; and
- extracting (102b) information on at least one pathology imaged on the at least one input radiology image, using the artificial intelligence module (53), which is trained to detect pathologies, and/or a statistical algorithm .

3. The method according to any of previous claims, further comprising, prior to generating (103) the initial radiology report, generating narrative descriptions of pathologies using a large language model or natural language processing module (54).

4. The method according to claim 1, further comprising, prior to generating (103) the initial radiology report, determining anatomy imaged on the at least one input radiology image and extracting information on pathologies imaged on the at least one input radiology image, and optionally generating narrative descriptions of pathologies using a vision language model (55).

5. The method according to any of previous claims, wherein the step of receiving (105) the feature pointer input comprises receiving an input via a image panel (220) by taking an action on image panel, wherein the action preferably includes pointing, zooming and measurements.

6. The method according to any of previous claims, comprising receiving (106b) the action identifier indicating addition of a feature from an artificial intelligence module and/or statistical algorithm (53) trained to detect pathologies.

7. The method according to any of previous claims, wherein the step of receiving (106a) the action identifier includes receiving a text description or voice description from the user through a speech recognition system.

8. The method according to any of previous claims, wherein the step of updating (107) the radiology report includes using an artificial intelligence large language model and/or natural language processing module (54) to automatically generate descriptive text based on the received feature pointer input.

9. The method according to any of previous claims, comprising presenting, via the user interface, a first text panel (255) with a short descriptive data related to the detected anatomies and pathologies and a second text panel (256) with a narrative descriptive data related to the detected anatomies and pathologies.

10. The method of claim 9, wherein a unique color is algorithmically assigned to each detected pathology and to each clinically relevant detected anatomy, and the same color is used concurrently to highlight the corresponding short descriptor in the first text panel, the corresponding narrative fragment in the second text panel, and at least one marker or region overlay in the image panel or panels.

11. The method according to any of previous claims, further comprising comparing (108) current radiology findings with previous studies and assembling these data when generating (109) a final radiology report.

12. The method according to any of previous claims, wherein the contents of the image panel (210, 220, 230, 240, 224) are correlated with the contents of the text panel (250, 255, 256) such that when a feature is selected on the image panel (210, 220, 230, 240, 224), a corresponding text related to that feature, if present, is highlighted on the text panel (250, 255, 256) and if a feature is selected on the text panel (250, 255, 256), a corresponding image fragment is highlighted on the image panel (210, 220, 230, 240, 224).

13. The method according to any of previous claims, further comprising, for each detected anatomy, calculating a relevance score that is a weighted combination of at least one of:
- spatial proximity of the anatomy to at least one of the detected pathologies;
- inclusion of the anatomy in a modality- or protocol-specific description of the examination;
- presence of the anatomy in a guideline-based lookup table of structures critical for the scanned region; and
- a user-defined preference value;
and wherein the step of generating the initial radiology report comprises inserting only those anatomies whose relevance score exceeds a predefined threshold.

14. The method of claim 1, wherein the at least one image panel displays the at least one input radiology image, and wherein each detected pathology or anatomy is rendered as a graphic overlay whose geometry is stored in image-space coordinates and is re-mapped in real time to displayed screen space whenever the user performs an image manipulation operation, so that the overlay of the pathology or anatomy remains spatially registered with the underlying input radiology image throughout such manipulations.

15. A computer-implemented radiology reporting system, comprising:
- at least one display device configured to concurrently render:
- an image panel displaying at least one input radiology image; and
- a text panel displaying descriptive data related to features detected in the at least one input radiology image;
- an input interface configured to receive user input from a user;
- a data storage that stores the at least one input radiology image and radiology reports; and
- a controller comprising at least one processor and a memory storing instructions that, when executed, cause the controller to:
- process the at least one input radiology image with at least one artificial-intelligence module (52, 53) to detect anatomies and pathologies and to generate, for each detected anatomy or pathology, corresponding positional data and descriptive data;
- automatically generate, prior to user review, an initial radiology report that aggregates, for every detected pathology and for at least one detected anatomy, the respective positional data and descriptive data;
- present the initial radiology report on the display device by:
- overlaying graphical markers derived from the positional data onto the image panel, and
- displaying the descriptive data in the text panel,
- wherein the image and text panels are dynamically linked so that selection of a feature in one panel highlights the corresponding feature in the other panel;
- receive, via the input interface and through a panel selected by a user, a feature-pointer input that designates a feature appearing in the image panel or the text panel;
- receive, in association with the feature-pointer input, an action identifier that indicates one of: addition of a feature, modification of a feature, or deletion of a feature; and
- update the radiology report by performing the action indicated by the action identifier with respect to the designated feature and refreshing the image and text panels so that they remain synchronized.

## Patentansprüche

1. Ein computerimplementiertes Verfahren zur Unterstützung eines Benutzers bei der Erstellung eines radiologischen Befundberichts aus mindestens einem eingegebenen radiologischen Bild, wobei das Verfahren umfasst:
- Verarbeiten (102, 102a, 102b) des mindestens einen eingegebenen radiologischen Bildes mit mindestens einem Modul für künstliche Intelligenz (52, 53), um Anatomien und Pathologien zu erkennen und Positionsdaten sowie beschreibende Daten in Bezug auf die erkannten Anatomien und Pathologien zu generieren;
- automatisches Erzeugen (103) eines ersten radiologischen Befundberichts vor der Überprüfung durch den Benutzer, wobei der Bericht für jede erkannte Pathologie und für mindestens eine erkannte Anatomie gesammelte Positionsdaten und beschreibende Daten in Bezug auf die erkannten Anatomien und Pathologien umfasst;
- Anzeigen (104) des ersten radiologischen Befundberichts über eine Benutzeroberfläche (20), die mindestens ein Bildfeld (210, 220, 230, 240, 224) umfasst, das das mindestens eine eingegebene radiologische Bild mit den erkannten Pathologien und der mindestens einen erkannten Anatomie, die darauf markiert sind, anzeigt, sowie ein Textfeld (250, 255, 256) mit beschreibenden Daten zu den erkannten Pathologien und der mindestens einen erkannten Anatomie;
- Ermöglichen der Überprüfung des ersten radiologischen Befundberichts durch den Benutzer, indem vom Benutzer eine Merkmalszeiger-Eingabe empfangen wird (105), die ein Merkmal auf dem Bildfeld (210, 220, 230, 240, 224) oder im Textfeld (250, 255, 256) markiert, wobei das Feld (210, 220, 230, 240, 224, 250, 255, 256), über das die Merkmalszeiger-Eingabe erfolgt, vom Benutzer auswählbar ist;
- Empfangen (106a, 106b) einer Aktionskennung, die auf das durch die Merkmalszeiger-Eingabe definierte Merkmal anzuwenden ist, wobei die Aktionskennung aus einer Gruppe ausgewählt wird, die Folgendes umfasst: Hinzufügen eines Merkmals, Ändern eines Merkmals und Löschen eines Merkmals; und
- Aktualisieren (107) des ersten radiologischen Befundberichts durch Ausführen der der empfangenen Aktionskennung entsprechenden Aktion in Bezug auf das durch die Merkmalszeiger-Eingabe definierte Merkmal und Anzeigen eines aktualisierten radiologischen Befundberichts über die Benutzeroberfläche (20).

2. Verfahren nach Anspruch 1, das ferner vor dem Erzeugen (103) des ersten radiologischen Befundberichts mindestens einen der folgenden Schritte umfasst:
- Extrahieren (101) von Informationen über die Diagnosetechnik des mindestens einen radiologischen Bildes unter Verwendung eines Moduls für künstliche Intelligenz und/oder eines statistischen Algorithmus (51);
- Bestimmen (102a) mindestens einer auf dem mindestens einen eingegebenen radiologischen Bild abgebildeten Anatomie unter Verwendung des Moduls für künstliche Intelligenz (52), das auf die Erkennung von Anatomien trainiert ist, und/oder eines statistischen Algorithmus; und
- Extrahieren (102b) von Informationen über mindestens eine auf dem mindestens einen eingegebenen radiologischen Bild abgebildete Pathologie unter Verwendung des Moduls für künstliche Intelligenz (53), das auf die Erkennung von Pathologien trainiert ist, und/oder eines statistischen Algorithmus.

3. Verfahren nach einem der vorstehenden Ansprüche, das ferner vor dem Erzeugen (103) des ersten radiologischen Befundberichts das Erzeugen narrativer Beschreibungen von Pathologien unter Verwendung eines großen Sprachmodells oder eines Moduls zur Verarbeitung natürlicher Sprache (54) umfasst.

4. Verfahren nach Anspruch 1, das ferner vor dem Erzeugen (103) des ersten radiologischen Befundberichts die Bestimmung der auf dem mindestens einen eingegebenen radiologischen Bild abgebildeten Anatomie und die Extraktion von Informationen über auf dem mindestens einen eingegebenen radiologischen Bild abgebildete Pathologien sowie optional das Erzeugen narrativer Beschreibungen von Pathologien unter Verwendung eines Bild-Sprachmodells (55) umfasst.

5. Verfahren nach einem der vorstehenden Ansprüche, wobei der Schritt des Empfangens (105) der Merkmalszeiger-Eingabe das Empfangen einer Eingabe über ein Bildfeld (220) durch Ausführen einer Aktion auf dem Bildfeld umfasst, wobei die Aktion vorzugsweise Zeigen, Zoomen und Messungen umfasst.

6. Verfahren nach einem der vorstehenden Ansprüche, umfassend das Empfangen (106b) der Aktionskennung, die das Hinzufügen eines Merkmals anzeigt, von einem Modul für künstliche Intelligenz und/oder einem statistischen Algorithmus (53), das bzw. der darauf trainiert ist, Pathologien zu erkennen.

7. Verfahren nach einem der vorstehenden Ansprüche, wobei der Schritt des Empfangens (106a) der Aktionskennung das Empfangen einer Textbeschreibung oder Sprachbeschreibung vom Benutzer über ein Spracherkennungssystem umfasst.

8. Verfahren nach einem der vorstehenden Ansprüche, wobei der Schritt des Aktualisierens (107) des radiologischen Befundberichts die Verwendung eines großen Sprachmodells der künstlichen Intelligenz und/oder eines Moduls zur Verarbeitung natürlicher Sprache (54) umfasst, um auf der Grundlage der empfangenen Merkmalszeiger-Eingabe automatisch beschreibenden Text zu generieren.

9. Verfahren nach einem der vorstehenden Ansprüche, umfassend das Anzeigen eines ersten Textfeldes (255) mit kurzen beschreibenden Daten zu den erkannten Anatomien und Pathologien sowie eines zweiten Textfeldes (256) mit narrativen beschreibenden Daten zu den erkannten Anatomien und Pathologien über die Benutzeroberfläche.

10. Verfahren nach Anspruch 9, wobei jeder erkannten Pathologie und jeder klinisch relevanten erkannten Anatomie algorithmisch eine eindeutige Farbe zugewiesen wird und dieselbe Farbe gleichzeitig verwendet wird, um den entsprechenden kurzen Beschreibungstext im ersten Textfeld, das entsprechende narrative Fragment im zweiten Textfeld und mindestens eine Markierung oder eine überlagerte Region im Bildfeld oder in den Bildfeldern hervorzuheben.

11. Verfahren nach einem der vorstehenden Ansprüche, ferner umfassend das Vergleichen (108) aktueller radiologischer Befunde mit früheren Untersuchungen und das Zusammenführen dieser Daten bei dem Erzeugen (109) eines endgültigen radiologischen Befundberichts.

12. Verfahren nach einem der vorstehenden Ansprüche, wobei der Inhalt des Bildfeldes (210, 220, 230, 240, 224) mit dem Inhalt des Textfeldes (250, 255, 256) so korreliert ist, dass bei Auswahl eines Merkmals im Bildfeld (210, 220, 230, 240, 224) ein entsprechender Text, der sich auf dieses Merkmal bezieht, sofern vorhanden, im Textfeld (250, 255, 256) hervorgehoben wird, und wenn ein Merkmal im Textfeld (250, 255, 256) ausgewählt wird, ein entsprechendes Bildfragment im Bildfeld (210, 220, 230, 240, 224) hervorgehoben wird.

13. Verfahren nach einem der vorstehenden Ansprüche, das ferner für jede erkannte Anatomie die Berechnung eines Relevanzwerts umfasst, der eine gewichtete Kombination aus mindestens einem der folgenden Elemente ist:
- räumliche Nähe der Anatomie zu mindestens einer der erkannten Pathologien;
- Einbeziehung der Anatomie in eine modalitäts- oder protokollspezifische Beschreibung der Untersuchung;
- Vorhandensein der Anatomie in einer auf Leitlinien basierenden Nachschlagetabelle von Strukturen, die für den gescannten Bereich kritisch sind; und
- einen benutzerdefinierten Präferenzwert;
und wobei der Schritt des Erzeugens des ersten radiologischen Befundberichts das Einfügen nur jener Anatomien umfasst, deren Relevanzwert einen vordefinierten Schwellenwert überschreitet.

14. Verfahren nach Anspruch 1, wobei das mindestens eine Bildfeld das mindestens eine eingegebene radiologische Bild anzeigt und wobei jede erkannte Pathologie oder Anatomie als grafische Überlagerung dargestellt wird, deren Geometrie in Bildraumkoordinaten gespeichert ist und in Echtzeit auf den angezeigten Bildschirmraum neu abgebildet wird, wann immer der Benutzer eine Bildmanipulationsoperation durchführt, so dass die Überlagerung der Pathologie oder Anatomie während solcher Manipulationen räumlich mit dem darunterliegenden eingegebenen radiologischen Bild registriert bleibt.

15. Ein computerimplementiertes radiologisches Berichtssystem, umfassend:
- mindestens eine Anzeigevorrichtung, die so konfiguriert ist, dass sie gleichzeitig darstellt:
- ein Bildfeld, das mindestens ein eingegebenes radiologisches Bild anzeigt; und
- ein Textfeld, das beschreibende Daten anzeigt, die sich auf in dem mindestens einen eingegebenen radiologischen Bild erkannte Merkmale beziehen;
- eine Eingabeschnittstelle, die so konfiguriert ist, dass sie Benutzereingaben von einem Benutzer empfängt;
- einen Datenspeicher, der das mindestens eine eingegebene radiologische Bild und radiologische Befundberichte speichert; und
- eine Steuereinheit, die mindestens einen Prozessor und einen Speicher umfasst, der Befehle speichert, die bei ihrer Ausführung die Steuereinheit veranlassen:
- das mindestens eine eingegebene radiologische Bild mit mindestens einem Modul für künstliche Intelligenz (52, 53) zu verarbeiten, um Anatomien und Pathologien zu erkennen und für jede erkannte Anatomie oder Pathologie entsprechende Positionsdaten und beschreibende Daten zu generieren;
- vor der Überprüfung durch den Benutzer automatisch einen ersten radiologischen Befundbericht zu erzeugen, der für jede erkannte Pathologie und für mindestens eine erkannte Anatomie die jeweiligen Positionsdaten und beschreibenden Daten zusammenfasst;
- den ersten radiologischen Befundbericht auf der Anzeigevorrichtung darzustellen, indem:
- aus den Positionsdaten abgeleitete grafische Markierungen auf das Bildfeld überlagert werden, und
- die beschreibenden Daten im Textfeld angezeigt werden,
- wobei das Bildfeld und das Textfeld dynamisch miteinander verknüpft sind, sodass die Auswahl eines Merkmals in einem Feld das entsprechende Merkmal im anderen Feld hervorhebt;
- über die Eingabeschnittstelle und über ein vom Benutzer ausgewähltes Feld eine Merkmalszeiger-Eingabe zu empfangen, die ein im Bildfeld oder im Textfeld erscheinendes Merkmal bezeichnet;
- in Verbindung mit der Merkmalszeiger-Eingabe eine Aktionskennung zu empfangen, die eines der folgenden angibt: Hinzufügen eines Merkmals, Ändern eines Merkmals oder Löschen eines Merkmals; und
- den radiologischen Befundbericht durch Ausführen der durch die Aktionskennung angegebenen Aktion in Bezug auf das bezeichnete Merkmal zu aktualisieren und das Bildfeld und das Textfeld aufzufrischen, sodass diese synchronisiert bleiben.

## Revendications

1. Procédé mis en œuvre par ordinateur destiné à assister un utilisateur dans la préparation d'un rapport radiologique à partir d'au moins une image radiologique d'entrée, le procédé comprenant les étapes consistant à :
- traiter (102, 102a, 102b) ladite au moins une image radiologique d'entrée à l'aide d'au moins un module d'intelligence artificielle (52, 53) afin de détecter des anatomies et des pathologies et de générer des données de position et des données descriptives relatives aux anatomies et pathologies détectées ;
- générer (103), de manière automatique et avant l'examen par l'utilisateur, un rapport radiologique initial comprenant, pour chaque pathologie détectée et pour au moins une anatomie détectée, des données de position collectives et des données descriptives relatives aux anatomies et pathologies détectées ;
- afficher (104) le rapport radiologique initial via une interface utilisateur (20) comprenant au moins un panneau d'image (210, 220, 230, 240, 224) affichant ladite au moins une image radiologique d'entrée avec les pathologies détectées et la ou les anatomies détectées marquées dessus et un panneau de texte (250, 255, 256) avec des données descriptives relatives aux pathologies détectées et à la ou aux anatomies détectées ;
- permettre à l'utilisateur de réviser le rapport radiologique initial, en recevant (105) de l'utilisateur une entrée de pointeur de caractéristique marquant une caractéristique sur le panneau d'image (210, 220, 230, 240, 224) ou sur le panneau de texte (250, 255, 256), le panneau (210, 220, 230, 240, 224, 250, 255, 256) par lequel l'entrée de pointeur de caractéristique est fournie étant sélectionnable par l'utilisateur ;
- recevoir (106a, 106b) un identifiant d'action à effectuer sur la caractéristique définie par l'entrée de pointeur de caractéristique, l'identifiant d'action étant sélectionné parmi un groupe comprenant : ajout d'une caractéristique, modification d'une caractéristique et suppression d'une caractéristique ; et
- mettre à jour (107) le rapport radiologique initial en effectuant l'action correspondant à l'identifiant d'action reçu par rapport à la caractéristique définie par l'entrée de pointeur de caractéristique et présenter un rapport radiologique mis à jour via l'interface utilisateur (20).

2. Procédé selon la revendication 1, comprenant en outre, avant de générer (103) le rapport radiologique initial, l'exécution d'au moins l'une des opérations suivantes consistant à :
- extraire (101) des informations sur la technique de diagnostic de ladite au moins une image radiologique, à l'aide d'un module d'intelligence artificielle et/ou d'un algorithme statistique (51) ;
- déterminer (102a) au moins une anatomie visible sur ladite au moins une image radiologique d'entrée, à l'aide du module d'intelligence artificielle (52), qui est entraîné à détecter des anatomies, et/ou d'un algorithme statistique ; et
- extraire (102b) des informations sur au moins une pathologie visible sur ladite au moins une image radiologique d'entrée, à l'aide du module d'intelligence artificielle (53), qui est entraîné à détecter des pathologies, et/ou d'un algorithme statistique.

3. Procédé selon l'une quelconque des revendications précédentes, comprenant en outre, avant de générer (103) le rapport radiologique initial, la génération de descriptions narratives des pathologies à l'aide d'un grand modèle de langage ou d'un module de traitement du langage naturel (54).

4. Procédé selon la revendication 1, comprenant en outre, avant de générer (103) le rapport radiologique initial, la détermination de l'anatomie visible sur ladite au moins une image radiologique d'entrée et l'extraction d'informations sur les pathologies visibles sur ladite au moins une image radiologique d'entrée, et, de manière facultative, la génération de descriptions narratives des pathologies à l'aide d'un modèle vision-langage (55).

5. Procédé selon l'une quelconque des revendications précédentes, dans lequel l'étape consistant à recevoir (105) l'entrée de pointeur de caractéristique comprend la réception d'une entrée via un panneau d'image (220) en effectuant une action sur le panneau d'image, l'action comprenant de préférence le pointage, le zoom et des mesures.

6. Procédé selon l'une quelconque des revendications précédentes, comprenant la réception (106b) de l'identifiant d'action indiquant l'ajout d'une caractéristique provenant d'un module d'intelligence artificielle et/ou d'un algorithme statistique (53) entraîné à détecter des pathologies.

7. Procédé selon l'une quelconque des revendications précédentes, dans lequel l'étape consistant à recevoir (106a) l'identifiant d'action comprend la réception d'une description textuelle ou vocale de la part de l'utilisateur via un système de reconnaissance vocale.

8. Procédé selon l'une quelconque des revendications précédentes, dans lequel l'étape consistant à mettre à jour (107) le rapport radiologique comprend l'utilisation d'un grand modèle de langage d'intelligence artificielle et/ou d'un module de traitement du langage naturel (54) pour générer automatiquement un texte descriptif sur la base de l'entrée de pointeur de caractéristique reçue.

9. Procédé selon l'une quelconque des revendications précédentes, comprenant la présentation, via l'interface utilisateur, d'un premier panneau de texte (255) contenant des données descriptives succinctes relatives aux anatomies et pathologies détectées et d'un second panneau de texte (256) contenant des données descriptives narratives relatives aux anatomies et pathologies détectées.

10. Procédé selon la revendication 9, dans lequel une couleur unique est attribuée de manière algorithmique à chaque pathologie détectée et à chaque anatomie détectée et cliniquement pertinente, et la même couleur est utilisée simultanément pour mettre en évidence le descripteur succinct correspondant dans le premier panneau de texte, le fragment narratif correspondant dans le second panneau de texte, et au moins un marqueur ou une superposition de région dans le ou les panneaux d'image.

11. Procédé selon l'une quelconque des revendications précédentes, comprenant en outre la comparaison (108) des résultats radiologiques actuels avec des études antérieures et l'assemblage de ces données lors de la génération (109) d'un rapport radiologique final.

12. Procédé selon l'une quelconque des revendications précédentes, dans lequel le contenu du panneau d'image (210, 220, 230, 240, 224) est corrélé avec le contenu du panneau de texte (250, 255, 256) de telle sorte que lorsqu'une caractéristique est sélectionnée sur le panneau d'image (210, 220, 230, 240, 224), un texte correspondant lié à cette caractéristique, s'il est présent, est mis en évidence sur le panneau de texte (250, 255, 256) et si une caractéristique est sélectionnée sur le panneau de texte (250, 255, 256), un fragment d'image correspondant est mis en évidence sur le panneau d'image (210, 220, 230, 240, 224).

13. Procédé selon l'une quelconque des revendications précédentes, comprenant en outre, pour chaque anatomie détectée, le calcul d'un score de pertinence qui est une combinaison pondérée d'au moins l'un des éléments suivants :
- la proximité spatiale de l'anatomie par rapport à au moins l'une des pathologies détectées ;
- l'inclusion de l'anatomie dans une description de l'examen spécifique à une modalité ou à un protocole ;
- la présence de l'anatomie dans une table de correspondance basée sur des lignes directrices et répertoriant les structures critiques pour la région scannée ;
- une valeur de préférence définie par l'utilisateur ;
et dans lequel l'étape consistant à générer le rapport radiologique initial comprend l'insertion uniquement des anatomies dont le score de pertinence dépasse un seuil prédéfini.

14. Procédé selon la revendication 1, dans lequel le au moins un panneau d'image affiche ladite au moins une image radiologique d'entrée, et dans lequel chaque pathologie ou anatomie détectée est représentée sous la forme d'une superposition graphique dont la géométrie est stockée en coordonnées de l'espace image et est remappée en temps réel dans l'espace écran affiché chaque fois que l'utilisateur effectue une opération de manipulation d'image, de sorte que la superposition de la pathologie ou de l'anatomie reste spatialement alignée avec l'image radiologique d'entrée sous-jacente tout au long de ces manipulations.

15. Système de rapport radiologique mis en œuvre par ordinateur, comprenant :
- au moins un dispositif d'affichage configuré pour afficher simultanément :
- un panneau d'image affichant au moins une image radiologique d'entrée ; et
- un panneau de texte affichant des données descriptives relatives à des caractéristiques détectées dans ladite au moins une image radiologique d'entrée ;
- une interface d'entrée configurée pour recevoir une entrée utilisateur provenant d'un utilisateur ;
- un stockage de données qui stocke ladite au moins une image radiologique d'entrée et des rapports radiologiques ; et
- un contrôleur comprenant au moins un processeur et une mémoire stockant des instructions qui, lorsqu'elles sont exécutées, amènent le contrôleur à :
- traiter ladite au moins une image radiologique d'entrée à l'aide d'au moins un module d'intelligence artificielle (52, 53) afin de détecter des anatomies et des pathologies et de générer, pour chaque anatomie ou pathologie détectée, des données de position et des données descriptives correspondantes ;
- générer automatiquement, avant l'examen par l'utilisateur, un rapport radiologique initial qui regroupe, pour chaque pathologie détectée et pour au moins une anatomie détectée, les données de position et les données descriptives respectives ;
- présenter le rapport radiologique initial sur le dispositif d'affichage en :
- superposant des marqueurs graphiques dérivés des données de position sur le panneau d'image, et
- affichant les données descriptives dans le panneau de texte,
- dans lequel les panneaux d'image et de texte sont liés de manière dynamique de sorte que la sélection d'une caractéristique dans un panneau met en évidence la caractéristique correspondante dans l'autre panneau ;
- recevoir, via l'interface d'entrée et par l'intermédiaire d'un panneau sélectionné par un utilisateur, une entrée de pointeur de caractéristique qui désigne une caractéristique apparaissant dans le panneau d'image ou le panneau de texte ;
- recevoir, en association avec l'entrée de pointeur de caractéristique, un identifiant d'action indiquant l'une des opérations suivantes : ajout d'une caractéristique, modification d'une caractéristique ou suppression d'une caractéristique ; et
- mettre à jour le rapport radiologique en effectuant l'action indiquée par l'identifiant d'action sur la caractéristique désignée et en actualisant les panneaux d'image et de texte de manière à ce qu'ils restent synchronisés.
